# EUROPEAN PATENT APPLICATION

(11) **EP 2 415 465 A1**
(43) Date of publication of application: **08.02.2012**
(21) Application number: 10757955.9
(22) Date of filing: 01.04.2010
(51) Int. Cl.: A61K 9/16, A61K 9/20, A61K 9/48, A61K 31/365, A61K 47/02, A61K 47/10, A61K 47/20, A61K 47/32, A61K 47/36, A61P 3/04

(54) **STABILISER-FREE PHARMACEUTICAL COMPOSITIONS OF TETRAHYDROLIPSTATIN**

(30) Priority: 03.04.2009 BR PI0901602
(71) Applicant: EMS S. A., 13186-901 Hortolândia - SP (BR)
(72) Inventor: TINTI, Flavia Giuliana, 13186-901 São Paulo - SP (BR); MATHA, Vladimir, 13186-901 São Paulo - SP (BR); MARQUES, Ricardo Vian, 13186-901 São Paulo - SP (BR)
(74) Representative: Ricker, Mathias
(86) International application number: PCT/BR2010/000097
(87) International publication number: WO 2010/111759

(57) **Abstract**

This invention refers to pharmaceutical compositions that comprise the mixture of an effective amount of leucine-derivative powders or granules and, at least, one pharmaceutically acceptable excipient substance used as carrier. In particular, the main feature of the pharmaceutical composition is free of preservative agents and might be formulated in any usual pharmaceutical oral formulation.

## Description

### INVENTION FIELD

This invention refers to pharmaceutical compositions that comprise the mixture of an effective amount of leucine-derivative powders or granules and, at least, one pharmaceutically acceptable excipient substance used as carrier and preservative-free, which can be formulated by any conventional oral pharmaceutical dosage form.

### INVENTION BASIS

Tetrahydrolipstatin compound (THL), also known as Orlistat (its generic name) or chemically by IUPAC as *N-*Formyl-L-leucine (1*S*)-1-[[(2*S*,3*S*)-3-hexyl-4-oxo-2-oxetanyl]methyl]dodecyl ester is a inhibitor of pancreatic lipases and usually used as an anti-obesity agent.

In view of the physicochemical characteristics of this compound, the active pharmaceutical ingredient requires special conditions of manipulation.

First of all, once THL presents melting point at 44° C, it is sensitive to thermal degradation. When THL is stored at dry atmosphere, its degradation starts around 40°C, and, in case of storage at wet environment, such degradation starts faster.

Secondly, considering its adherence behavior, THL cannot be easily formulated by mixing wet granule/powder, making its manipulation more difficult in hard gelatin tablets or capsules.

In view of the compound sensitivity to humidity and heat, the technique lacks stable THL formulations, which avoid compound degradation to humidity and heat caused during the production process and during the storage of drugs with such a compound as active pharmaceutical ingredient. Furthermore, formulations that mitigate poor flow and adherence phenomena, providing, thus, good conditions for usual pharmaceutical oral forms, such as hard gelatin capsules or tablets are difficult to reach.

Several studies can be found at specialized literature on THL compound, as follows:
The US patent US 4, 598, 089 discloses new leucine-derivative compounds as conventional pharmaceutical oral forms for pancreatic lipase inhibition. They can be used for control and prevention of obesity and hyperlipidemia. THL compounds are difficult to formulate due to its adherence phenomena.

The patent US 6, 004, 996 describes the compound instability, by suggesting pharmaceutical compositions of THL pellets prepared for extrusion and spheronization when they are in the presence of preservatives, in order to overcome the technical problems. More specifically, the referred US document describes agglomerated particles between 0.25 mm and 2.0 mm in diameter with THL prepared for pelletizing, comprising wetting agents that are mixed with THL and excipients, where the particles are prepared in the extruder, followed by the spheronization and drying process.

The patents EP 1 399 153 and EP 1 216 025 describe formulations on which the use of fatty acids or specific surfactant classes interfere on physicochemical solubility properties of the active pharmaceutical ingredient, allowing the formulations of such compound at higher concentrations or non-solid carriers.

The patent US 6, 703, 369 discloses THL pharmaceutical compositions using fatty acid ester-derivative products as preservatives.

The present invention surprisingly has achieved stable THL pharmaceutical compositions without preservative agents.

### INVENTION SUMMARY

This invention refers to pharmaceutical compositions that comprise the mixture of an effective amount of leucine-derivative in dosage forms such as powders or granules and, at least, one pharmaceutically acceptable excipient substance used as carrier. Specifically the object of this invention is a pharmaceutical composition free of preservative agents. In addition, this invention provides agglomerated or powders that can achieve stability in any oral pharmaceutical composition. Preferably, hard gelatin capsules or tablets are used for the preparation of drugs.

### DETAILED DESCRIPTION OF THE INVENTION

This invention refers to pharmaceutical compositions that comprise the mixture of an effective amount of leucine-derivative powders or granules and, at least, one pharmaceutically acceptable excipient substance used as carrier.

More specifically, in the compositions of the present invention the leucine derivative used as active pharmaceutical ingredient is the Tetrahydrolipstatin (THL).

Particularly, in this invention, the main feature is pharmaceutical compositions free of preservative agents.

This invention provides agglomerated or powders that can achieve stability in any oral pharmaceutical composition. Preferably, agglomerated or powders are used for the preparation of hard gelatin capsules or tablets.

The term "free of preservative agents" means absence of excipients that provide stability to the active pharmaceutical ingredient or, in other words, the THL stability. More specifically, the terminology "preservative" must be understood as an agent with humidity absorption rate higher than active pharmaceutical ingredient one, or, in other words, higher than THL.

The referred pharmaceutical composition has a active pharmaceutical ingredient of approximately 20-60% in weight. More specifically, the formulation has also one or more glidant, solvent, surfactant, lubricant and dispersant agents or their mixtures.

In particular, such a rate of excipients is approximately between 1 and 10% of glidant agent, 5 and 60% of diluting agent, 1 and 5% of surfactant agent and 2 and 3% of lubricant agent and about 5 and 10% of disintegrant agent.

Among the excipient substances, the diluents are selected from pregelatinized starch or mannitol.

The disintegrants are selected from the group with sodium starch glycolate, cross-linked PVP, croscarmellose sodium, crospovidone or low-substituted HPC.

The surfactant is selected from the group of sodium dodecyl sulfate, polyoxyethylene, sodium lauryl ether sulfate, sodium lauryl etoxysulfate and sodium polyoxyethylene lauryl sulfate.

The glidant agent is a dry lubricant material, such as colloidal silicon dioxide, calcium silicate, magnesium silicate (commonly known as talc), precipitated silica.

The preferable pharmaceutical composition of this invention, has approximately 40% of THL, 45% of pregelatinized starch, 5% of silicon dioxide, 3% sodium lauryl sulfate, 5% of crospovidone, and 2% of talc. However, it is worth to highlight that such concentrations are not limited to the exposed in this invention.

In general, the pharmaceutical composition in granulated form can be preferably produced by means of the process as follows:
- Add into a granulator the active pharmaceutical ingredient, the disintegrant, the surfactant and the glidant agent;
- Granulate the powder mixture with a solvent and take to the drying process;
- Add the remainder of the disintegrant, the surfactant and the glidant agent to the dried granulated material until obtaining a homogeneous mixture; and
- Compression or encapsulation of the final mixture.

Among the solvents that can be used in this process, it is possible to list an alcohol, such as ethanol, water or a mixture of both. However, the pharmaceutical compositions are not limited to a single process of production.

It is possible to prepare the pharmaceutical compositions by simply mixing the substances under powder form as follows:
- Add into a mixer the active pharmaceutical ingredient to the glidant agent, the diluent and, then, mix them until homogenizing;
- Afterwards, add the disintegrant and the surfactant, and then mix them until homogenizing; and finally
- Encapsulate or compress the mixture

Unexpectedly the pharmaceutical compositions (mixed and / or granulated THL) with the described excipients provide excellent protection against humidity.

Besides stabilizing the formulation, the granulated and/or mixed THL also minimizes the adherence effects, which consist of a phenomenon frequently encountered during the formulation of THL. Thus, an easy manipulation of tablets by direct compression and/or wet granulation or encapsulation is provided, in addition to good technological parameters, in order to produce oral pharmaceutical forms, such as hard gelatin capsules or tablets.

The mixtures prepared to obtain pharmaceutical compositions are stable and they can be used for filling hard gelatin capsules without adherence.

The substantially preferred advantage of this invention is that THL is not exposed to humidity increase or higher temperatures throughout the process, reducing the likelihood of thermal degradation.

Another advantage is that the obtained pharmaceutical composition reduce substantially the adherence and poor flow phenomena, making it ideal for dosage form in hard gelatin capsules or tablets.

For a better understanding on the invention, the present invention will now be illustrated by the following examples. It is understood, however, that such examples are provided for illustration only, and the invention is not intended to be limited by the examples. The formulation based on the system employed in the examples can be formed by any suitable method known in the art.

### EXAMPLE 1: PARTICULATE PHARMACEUTICAL FORMULATIONS:

**Table 1: Concentrations**

| Compounds | % (p/p) |
|---|---|
| Orlistat | 40.00 |
| Pregelatinized | 45.00 |
| starch | |
| Colloidal Silicon | 5.00 |
| Dioxide | |
| Crospovidone | 5.00 |
| Sodium lauryl | 3.00 |
| sulfate | |
| Talc | 2.00 |

Regarding the Example 1, the formulations and pharmaceutical compounds are produced through the process as follows:
1. Add Orlistat, pregelatinized starch, sodium lauryl sulphate, a first part of a crospovidone and a first part of the silicon dioxide into the granulator;
2. Granulate the powder mixture with ethanol;
3. Dry it at approximately 30°C and calibrate the dry granulate;
4. Add the remainder of the crospovidone, the silicon dioxide and the talc to the dry granule, and then mix them; and
5. Encapsulate or compress them.

### EXAMPLE 2: PARTICULATE PHARMACEUTICAL FORMULATIONS:

**Table 2: Concentrations**

| Compounds | % |
|---|---|
| Orlistat | 40.00 |
| Pregelatinized | 40.00 |
| starch | |
| Colloidal Silicon | 5.00 |
| Dioxide | |
| Crospovidone | 10.00 |
| Sodium lauryl | 3.00 |
| sulfate | |
| Talc | 2.00 |

Regarding the Example 2, the formulations and pharmaceutical compounds are produced through the process as follows:
1. Add Orlistat, pregelatinized starch, sodium lauryl sulphate, a first part of a crospovidone and a first part of the silicon dioxide into the granulator;
2. Granulate the powder mixture with ethanol;
3. Dry it at approximately 30°C and calibrate the dry granulate;
4. Add the remainder of the crospovidone, the silicon dioxide and the talc to the dry granule, and then mix them
5. Encapsulate or compress as per instructions

### EXAMPLE 3: SIMPLE MIXTURE PROCESS:

**Table 3: Concentrations**

| Compounds | % |
|---|---|
| Orlistat | 40.00 |
| Pregelatinized starch | 5.00 |
| Colloidal Silicon Dioxide | 5.00 |
| Crospovidone | 2.00 |
| Sodium lauryl sulfate | 3.00 |
| Talc | 2.00 |
| Mannitol | 43.00 |

Regarding the example 3, the formulations and pharmaceutical compounds are produced through the process as follows:
1. Add Orlistat, mannitol and silicon dioxide to the mixer and then mix them;
2. Then, add pregelatinized starch, crospovidone and sodium lauryl sulphate and mix them;
3. Add talc and homogenize it; and
4. Encapsulate or compress the mixture as per specifications.

### EXAMPLE 4: RESULTS OF STABILITY TESTS:

The pharmaceutical compositions obtained in the aforementioned examples were exposed to temperature conditions of approximately 25° C and humidity of 60% over a period of about six months. Evaluations on stability of the formulations were carried out on a periodical basis. The results for each studied period are presented in Table 4. These results proves the stability of the new proposed pharmaceutical compositions according to the methodology described in "A stability-indicating high performance liquid chromatographic assay for the determination of orlistat in capsules, A. Mohammadi et al. / J. Chromatogr. A 1116 (2006) 153-157*".*

**Table 4: Orlistat stability data**

| Example | Orlistat percentage | | |
|---|---|---|---|
| | 1 month | 3 months | 6 months |
| 1 | 99.0 | 98.6 | 98.0 |
| 2 | 99.2 | 98.8 | 97.8 |
| 3 | 100.0 | 99.2 | 98.4 |

The invention described herein is not limited to that achievement, and those with know-how on the technique will realize that any particular characteristic introduced in this invention should be understood only as something described to ease the understanding and cannot be made without departing from the described original concept. The limiting characteristics of the object concerning this invention relate to the claims that are part of this report.

## Claims

1. Pharmaceutical compound **characterized by** a mixture of leucine-derivative compound powders or granules and, at least, one pharmaceutically acceptable excipient substance used as carrier.

2. Pharmaceutical compound according to the claim 1, **characterized by** a specific leucine-derivative compound: The Tetrahydrolipstatin (THL).

3. Pharmaceutical compound according to the claim 1, **characterized by** being free of preservative agents.

4. Pharmaceutical compound according to the claim 1, **characterized by** the presence of THL, glidant agent, diluent, surfactant, lubricant and dispersant.

5. Pharmaceutical compound according to the claim 4, **characterized by** diluents selected among pregelatinized starch or mannitol.

6. Pharmaceutical compound according to the claim 4, **characterized by** dispersants selected among the group of sodium starch glycolate, cross-linked PVP, croscarmellose sodium, crospovidone or low-substituted HPC.

7. Pharmaceutical compound according to the claim 4, **characterized by** the surfactant selected among the group of sodium dodecyl sulfate, polyoxyethylene, sodium lauryl ether sulfate, sodium lauryl etoxysulfate and sodium polyoxyethylene lauryl sulfate.

8. Pharmaceutical compound according to the claim 4 which the glidant agent is colloidal silicon dioxide.

9. Pharmaceutical compound according to claim 4, **characterized by** the above-mentioned colloidal silicon dioxide being talc.

10. Pharmaceutical compound according to claim 1 with characteristics of being a possible oral pharmaceutical formulation.

11. Pharmaceutical compound according to claim 1, **characterized by** being used in the preparation of medicines in hard gelatin capsules without adherence or pills.

12. Pharmaceutical formulation for the production of the pharmaceutical compound claimed between item 1 and 11, **characterized by** being free of preservative agents and weighing approximately 20-60% of THL, 1-10% of glidant agent 1-5% of surfactant, 2-3% of lubricant and 5-10% of dispersant.

13. Pharmaceutical formulation according to claim 12, **characterized by** the diluents selected among pregelatinized starch or mannitol.

14. Pharmaceutical formulation according to the claim 12, **characterized by** the dispersants selected among the group of sodium starch glycolate, cross-linked PVP, croscarmellose sodium, crospovidone or low-substituted HPC.

15. Pharmaceutical formulation according to the claim 12, **characterized by** the surfactant selected among the group of sodium dodecyl sulfate, polyoxyethylene, sodium lauryl ether sulfate, sodium lauryl etoxysulfate and sodium polyoxyethylene lauryl sulfate.

16. Pharmaceutical formulation according to the claim 12, which the glidant agent is colloidal silicon dioxide.

17. Pharmaceutical formulation according to the claim 12 which the above-mentioned colloidal silicon dioxide is talc.

18. Pharmaceutical formulation according to the claim 12, **characterized by** THL preferably weighing approximately 40%, pregelatinized starch weighting approximately 45%, silicon dioxide weighting approximately 5%, sodium lauryl sulfate of approximately 3%, crospovidone of approximately 5% and talc 2%.

19. Pharmaceutical formulation according to claim 12, **characterized by** the possibility of being available in pharmaceutical oral forms.

20. Pharmaceutical formulation according to the claim 12, **characterized by** being hard gelatin capsules or pills without adherence.
